(19) Europäisches Patentamt
European Patent Office
Office européen des brevets

(11) Publication number: **0 127 216 B1**

(12) **EUROPEAN PATENT SPECIFICATION**

(45) Date of publication of patent specification: **02.01.92**

(51) Int. Cl.[5]: **C07J 13/00**, C07J 41/00, C07J 43/00

(21) Application number: **84200596.9**

(22) Date of filing: **27.04.84**

(54) **Process for the preparation of 21-hydroxy-20-keto-delta16-steroids and intermediate compounds formed in this process.**

(30) Priority: **29.04.83 EP 83200618**

(43) Date of publication of application:
**05.12.84 Bulletin 84/49**

(45) Publication of the grant of the patent:
**02.01.92 Bulletin 92/01**

(84) Designated Contracting States:
**AT BE CH DE FR GB IT LI LU NL SE**

(56) References cited:
**EP-A- 0 023 856**
**EP-A- 0 058 097**
**DE-A- 1 906 560**
**DE-C- 927 029**
**FR-A- 1 555 991**
**FR-A- 1 568 712**
**GB-A- 2 086 907**
**US-A- 2 963 496**
**US-A- 3 056 730**

**CHEMICAL ABSTRACTS, vol. 91, no. 19, 5th November 1979, page 95, no. 151730t, Columbus, Ohio, US; A.V. KAMERNITSKII et al.: "Biological activity of transformed steroids. 11. Relation between the degree of steroid molecule flatness and mineralocorticoid activity"**

(73) Proprietor: **ROUSSEL-UCLAF**
**35, Boulevard des Invalides**
**F-75007 Paris(FR)**

(72) Inventor: **Van Leusen, Albert Mattheus**
**De Savornin Lohmanlaan 23**
**NL-9722 HC Groningen(NL)**
Inventor: **Van Leusen, Adriaan Marinus**
**Binnensingel 8**
**NL-9951 GD Winsum(NL)**

(74) Representative: **Vieillefosse, Jean-Claude et al**
**Département des Brevets ROUSSEL-UCLAF**
**111 Route de Noisy B.P. 9**
**F-93230 Romainville(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 127 216 B1

**JOURNAL OF THE AMERICAN CHEMICAL SOCIETY, vol. 77, no. 4, 28th February 1955, pages 1028-1032, Washington, D.C., US; W.S. ALLEN et al.: "Steroidal cyclic ketals. XII. The preparation of delta16-steroids"**

**JOURNAL OF ORGANIC CHEMISTRY, vol. 44, no. 9, September 1979, pages 1582-1584, Washington, D.C., US; V. VanRHEENEN et al.: "New synthesis of cortico steroids from 17-keto steroids: application and stereochemical study of the unsaturated sulfoxide-sulfenate rearrangement"**

## Description

The invention relates to a new process for the preparation of 21-hydroxy-20-keto-steroids from 17-(isocyano-sulfonyl-methlylene)-steroids whereby simultaneously an unsaturated bond is introduced between $C_{16}$ and $C_{17}$. Furthermore, the invention relates to new intermediate compounds formed in this process and their preparation.

Steroids are used on a large scale as constituents of many kinds of pharmaceutical compositions. Dependent on the substituent pattern of the carbon-skeleton the steroids can be divided into a number of main classes. An important main class of steroids is formed by the corticosteroids. The natural representatives of the corticosteroids are usually produced by the adrenal gland. Corticosteroids are characterised by the presence of a 3-keto group, a delta-4 bond, an 11-beta-hydroxyl group, a 17-alpha-hydroxyl group and a 17-beta-hydroxyacetyl side chain.

During a long time corticosteroids were made by chemical degradation of gall acids as cholic acid, desoxycholic acid and glycocholic acid. Afterwards, hecogenin, which could be isolated from plants, particularly from numerous Agave species, became an important raw material too. Since the possibility of introduction of an 11-hydroxy group by microbiological methods, diosgenin, which could be isolated from numerous Dioscoreacaea species, and stigmasterol, usually isolated from the phytosterol mixture from soya or calabar beans, have become the most important raw material for the preparation of corticosteroids.

Much attention has been given to new, cheaper raw materials for the synthesis of pharmaceutically active steroids. Therefore, the degradation of the abundant soya bean derived sterols sitosterol and campesterol by a microbiological method into 17-oxo steroids was extensively investigated. As a result thereof 17-oxo steroids are readily available now at low prices which makes these compounds, together with the possibility of the introduction of an 11-hydroxy group by microbiological methods, ideal starting materials for corticosteroid syntheses.

A number of chemical syntheses for the construction of the corticosteroid side chain from 17-oxo steroids is known. For instance in J. Org. Chem., 44, 1582 (1979) a method is described which uses a sulfenate-sulfoxide rearrangement for the introduction of the 17-(dihydroxyacetone) side chain.

Another route is described in J.C.S. Chem. Comm., 1981, 775, in which the reaction of 17-oxo steroids with ethyl isocyanoacetate is described, followed by a number of other reactions, which ultimately result in the dihydroxyacetone side chain of corticosteroids. Other syntheses of the corticosteroid side chain or of compounds which can be used as precursors therefore are described in J.C.S. Chem. Comm., 1981, 774, J.C.S. Chem. Comm., 1982, 551, Chem. Ber., 113, 1184 (1980) and J. Org. Chem., 1982, 2993.

The present invention relates to a new process for the introduction into steroids of a 17-side chain containing a 21-hydroxy group and a 20-keto group under the simultaneous formation of an unsaturated bond between $C_{16}$ and $C_{17}$.

The invention provides a process for the preparation of 21-hydroxy-20-keto-delta$^{16}$ steroids of formula III:

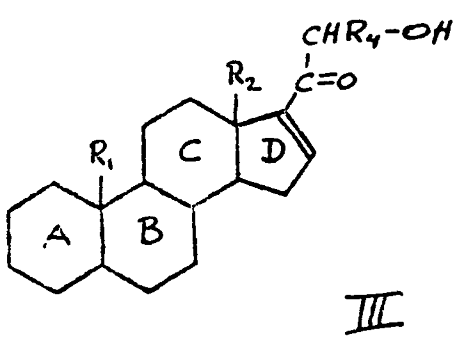

III

in which $R_1$ represents a hydrogen atom or an alkyl group having 1 to 4 carbon atoms, or is absent in the case of a double bond between $C_{10}$ and $C_1$, $C_5$ or $C_9$,
$R_2$ represents a hydrogen atom or an alkyl group having 1 to 4 carbon atoms,
$R_4$ represents a hydrogen atom or an alkyl group having 1 to 4 carbon atoms and optionally substituted by one or more halogen atoms or alkoxy groups or a phenyl group optionally substituted by one or more halogen atoms or alkyl or alkoxy groups
and the rings A, B, C and D may contain one or more double bonds and may be substituted by one or

more of the following groups: hydroxy, amino, oxo, halogen, alkyl, alkylene, alkoxy, alkoxyalkoxy, epoxy, methylene, alkylenedioxy, alkylenedithio or alkyleneoxythio by reacting a 17-(isocyano-sulfonyl-methylene)-steroid of formula I:

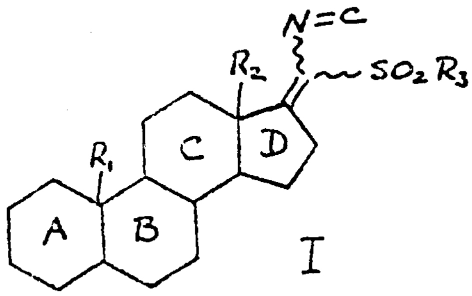

I

in which $R_1$ and $R_2$ have the meaning as listed above and rings A, B, C and D may contain double bonds and may carry substituents as defined above and in which $R_3$ respresents an alkyl group, an aryl group substituted or not substituted by one or more halogen atoms or alkyl, alkoxy, nitro or cyano groups or represents a dialkylamino group, wherein the alkyl groups together with the nitrogen atom may form a heterocyclic ring with up to 8 ring atoms and optionally containing an oxygen atom under basic conditions with an aldehyde $R_4HO$, where

$R_4$ represents a hydrogen atom or an alkyl group having 1 to 4 carbon atoms and optionally substituted by one or more halogen atoms or alkoxy groups or a phenyl group optionally substituted by one or more halogen atoms or alkyl or alkoxy groups and with an alkyl alcohol or an arylalkyl alcohol. and followed by hydrolysis of the intermediate 17-(2-$OR_5$-3-oxazolin-4-yl)-delta$^{16}$ steroids, in which $OR_5$ is alkoxy or arylalkoxy.

The invention comprises also a process for the preparation of 17-(2-$OR_5$-3-oxazolin-4-yl)-delta$^{16}$steroids of formula II

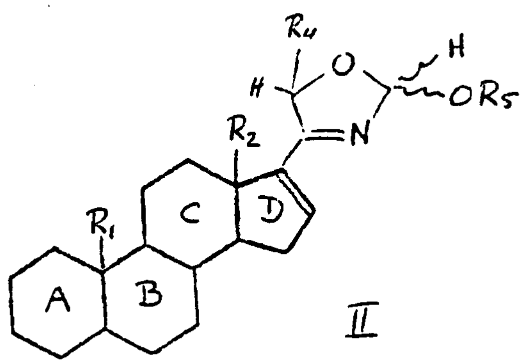

II

in which $R_1$, $R_2$, $R_4$ and $R_5$ have the meanings as defined above, rings A, B, C and D may contain double bonds and may carry substituents as defined above, by reacting a 17-(isocyano-sulfonyl-methylene) steroid as defined above under basic conditions with an aldehyde and an alcohol as defined above.

The invention comprises further the new 17-(2-$OR_5$-3-oxazolin-4-yl)-delta$^{16}$ steroids (II) in which $R_1$ represents a hydrogen atom or a methyl group or is absent in the case of a double bond between $C_{10}$ and $C_1$, $C_5$, or $C_9$, $R_2$ represents a hydrogen atom or a methyl group, $R_4$ represents a hydrogen atom or an alkyl or a phenyl group, $R_5$ represents an alkyl, group, and the rings A, B,C, and D optionally contain one ore more double bonds, and are optionally substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups or alkylenedioxy, alkylenedithio or alkyleneoxythio groups.

The preparation of the above-mentioned 17-(isocyano-sulfonyl-methylene) steroids is disclosed in the simultaneously filed application No. 84200595 (EP-A-0124934), entitled: "17-(Isocyano-sulfonyl-methylene) steroids, 17-(formamido-sulfonyl-methylene) steroids and their preparation", the contents of which have to be regarded as included herein. In this application a process is described for the introduction of a 17-(isocyano-sulfonyl-methylene)-group into a 17-oxo-steroid.

The 21-hydroxy-20-keto-delta[16] steroids obtained according to the present invention can be used for the preparation of pharmaceutically active compounds, especially corticoid steroids, by methods known in the art.

Suitable 17-(isocyano-sulfonyl-methylene) steroids for the process of the invention have the general formula

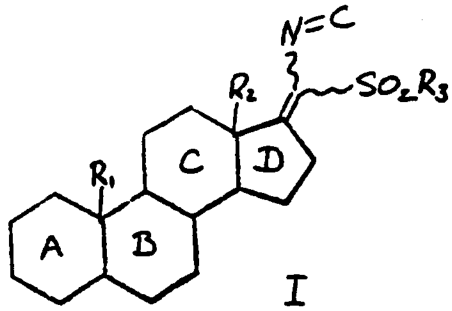

I

in which $R_1$ represents a hydrogen atom or an alkyl group having 1 to 4 carbons, preferably a methyl group, or is absent in the case of a double bond between $C_{10}$ and $C_1$, $C_5$ or $C_9$, $R_2$ represents a hydrogen atom or an alkyl group having 1 to 4 carbons, preferably a methyl group, $R_3$ represents an alkyl or dialkylamino group or an aryl group substituted or not substituted by one or more halogen atoms or alkyl, alkoxy, nitro or cyano groups, and the rings A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, alkylenedithio or alkyleneoxythio groups.

When $R_3$ represents an alkyl group, suitable alkyl groups are straight or branched alkyl groups having 1 to 10 carbon atoms.

When $R_3$ represents a dialkylamino group, suitable dialkylamino groups are dialkylamino groups wherein the alkyl groups are the same or different and contain 1-8 carbon atoms, preferably 1-4 carbon atoms, or a dialkylamino group wherein the alkyl groups together with the nitrogen atom form a heterocyclic ring which optionally may contain an oxygen atom, the ring containing up to 8 ring atoms. Preferred dialkylamino groups are dimethylamino, diethylamino, pyrrolidino and morpholino.

When $R_3$ represents an aryl group, suitable groups are phenyl and naphtyl groups, substituted or not substituted by a halogen atom, one or more alkyl groups or an alkoxy group, preferably a phenyl, p-methoxyphenyl or p-methylphenyl group.

When the rings A, B, C and D contain one or more double bonds, these double bonds are preferably present between $C_1$ and $C_2$, $C_3$ and $C_4$, $C_4$ and $C_5$, $C_5$ and $C_6$, $C_6$ and $C_7$, $C_9$ and $C_{10}$, $C_9$ and $C_{11}$ and/or $C_{11}$ and $C_{12}$. More preferably the double bond is present between $C_4$ and $C_5$ and/or $C_9$ and $C_{11}$.

When two or more double bonds are present especially the following systems are preferred: $C_3$-$C_4$ and $C_5$-$C_6$, $C_4$-$C_5$ and $C_6$-$C_7$, $C_1$-$C_2$ and $C_4$-$C_5$, $C_1$-$C_2$, $C_3$-$C_4$ and $C_5$-$C_{10}$ and $C_1$-$C_2$, $C_4$-$C_5$ and $C_6$-$C_7$. Preferably there is also a double bond between $C_9$ and $C_{11}$.

When the rings A, B, C and D are substituted by a hydroxy group, suitable groups are 3-, 9-, 11-, 12- or 14-hydroxy groups, preferably a 3- or 9-hydroxy group.

When the rings A, B, C and D are substituted by an amino group, suitable amino groups are 3-alkylamino groups, preferably containing 1-4 carbon atoms, 3-dialkylamino groups wherein the alkyl groups are the same or different, each alkyl group preferably containing 1-4 carbon atoms, or amino groups in which the nitrogen atom together with the alkyl groups forms a heterocyclic ring, preferably containing 1-8 ring atoms, which ring optionally may contain an oxygen atom. Particularly preferred are dimethylamino, diethylamino, pyrrolidino and morpholino.

When the rings A, B, C and D are substituted by an oxygen atom this oxygen atom is preferably present at $C_3$, $C_{11}$ or $C_{12}$.

When the rings A, B, C and D are substituted by a halogen atom, suitable halogen atoms are 6-, 9- or 11-fluorine, chlorine or bromine atoms, preferably 6- or 9-fluorine or chlorine atoms.

When the rings A, B, C and D are substituted by an alkyl group, suitable alkyl groups are 1-, 2-, 6-, 7- or 16-methyl groups, preferably 1- or 6-methyl.

When the rings A, B, C and D are substituted by an alkoxy group, suitable alkoxy groups are 3-, 9-, 11-

or 12-alkoxy groups containing 1-4 carbon atoms, preferably 3-, 9-, or 11-methoxy or ethoxy groups.

When the rings A, B, C and D are substituted by an alkoxyalkoxy group, suitable groups are 3- or 11-methoxymethoxy, methoxyethoxy or tetrahydropyranyloxy.

When the rings A, B, C and D are disubstituted, suitable substituents are epoxy groups at $C_1$ and $C_2$ or $C_9$ and $C_{11}$ or a methylene group attached to $C_1$ and $C_2$ or a 3,3-alkylenedioxy, a 3,3-alkylenedithio or a 3,3-alkyleneoxythio group. The alkylene group preferably contains 2 or 3 carbon atoms.

More particularly the invention relates to compounds in which $R_1$ and $R_2$ represent methyl or in which $R_1$ is absent, which are substituted by halogen, especially fluorine, or hydroxy at $C_9$ and a hydroxy or keto group at $C_{11}$, or which contain functional groups, as a double bond or epoxy group between $C_9$ and $C_{11}$, which can be converted by methods known in the art into the groups mentioned before, and which contain a keto group at $C_3$ and double bonds between $C_1$ and $C_2$ and/or $C_4$ and $C_5$, or which contain functional groups which can be converted into the keto group and double bonds mentioned before.

The invention also relates to the intermediate 17-(2-$OR_5$-3-oxazolin-4-yl)-delta$^{16}$ steroids of the general formula II as defined above and in which $R_4$ represents a hydrogen atom or a alkyl or phenyl group, but preferably a hydrogen atom and $R_5$ represents an alkyl group, preferably a methyl group and to a process to prepare these compounds by reacting a 17-(isocyano-sulfonyl-methylene) steroid with an aldehyde and an alcohol. As the 17-(2-$OR_5$-3-oxazolin-4-yl)-delta$^{16}$ steroids can be isolated and hydrolyzed later on, the invention also comprises the preparation of 21-hydroxy-20-keto-delta$^{16}$ steroids as defined above characterized by hydrolysis of the corresponding 17-(2-$OR_5$-3-oxazolin-4-yl)-delta$^{16}$ steroids. It is remarked however, that it is preferred that the two reactions are combined to a "one-pot-process".

The reaction of 17-(isocyano-sulfonyl-methylene)steroids with an aldehyde and an alcohol is carried out under basic conditions.

The reaction can be carried out in an inert organic solvent to which a base is added. Suitable organic solvents are methylene chloride, chloroform, 1,2-dichloroethane, benzene, toluene, chlorobenzene, dioxan, bis-(2-methoxyethyl)ether, 1,2-dimethoxyethane, tetrahydrofuran or mixtures thereof. As the first step and the second step of may be performed in a one-pot-reaction preferably a solvent is used in which also the hydrolysis can be carried out.

Suitable bases which can be used are metal hydroxides and quaternary ammonium and phosphonium hydroxides, preferably alkali hydroxides, e.g. potassium hydroxide, and quaternary ammonium hydroxides, e.g. triethylbenzylammonium hydroxide. Alkali alcoholates, e.g. potassium-butoxide, may be used. The reaction can be carried out at a temperature between 0° and 100° C, preferably between 0° an 30° C. Sometimes it may be necessary to add a catalyst to the reaction mixture in the form of a quaternary ammonium or phosphonium salt, for instance a trimethylbenzyl ammonium halogenide, a triethylbenzyl ammonium halogenide, a tetrabutyl ammonium halogenide or an alkyl triaryl phosphonium halogenide. Also crown ethers, as 15-crown-5 or 18-crown-6, can be used.

Suitable reaction conditions are also phase transfer-conditions, i.e. a two phase system of an organic layer and an aqueous layer to which an amount of a phase transfer catalyst salt has been added.

The reaction can be carried out at temperatures between 0° and 100° C, preferably between 0° and 30° C. See for a general survey of phase-transfer reactions E.V. Dehmlov and S.S. Dehmlov, Phase Transfer Catalysis, Weinheim Verlag Chemie, 1980.

Suitable organic solvents for the organic layer are methylene chloride, chloroform, 1,2-dichloroethane, benzene, toluene, chlorobenzene and dichlorobenzene. In general all those organic solvents can be used which are immiscible with water, and in which the relevant compounds are soluble.

Suitable aqueous layers are solutions of alkali metal hydroxides in water, for example a 5-50% solution of lithium hydroxide, sodium hydroxide or potassium hydroxide. Suitable phase transfer catalysts are quaternary ammonium and phosphonium salts and crown-ethers, for instance trimethylbenzyl ammonium halogenides, triethyl benzyl ammonium halogenides, tetrabutyl ammonium halogenides, alkyl triaryl phosphonium halogenides, 15-crown-15 and 18-crown-6.

Examples of aldehydes wich can be used in the reaction are arylaldehydes, alkylaldehydes and formaldehyde. Suitable aldehydes are phenylaldehydes in which the phenyl group optionally is substituted by one or more halogen atoms or alkyl or alkoxy groups, alkyl aldehydes in which the alkyl group has 1-4 carbon atoms and optionally is substituted by one or more halogen atoms or alkoxy groups. Preferred aldehydes are benzaldehyde, acetaldehyde and formaldehyde, more particularly formaldehyde. The formaldehyde is used either as formaline (solution in water), as paraformaldehyde or as trioxan.

Suitable alcohols are alkyl alcohols and arylalkyl alcohols. Preferred alcohols are $C_1$-$C_6$ alkyl alcohols, as methanol and ethanol. A preferred arylalkyl alcohol is benzyl alcohol. In general all alcohols can be used which do not interfere in the reaction. Preferably methanol or ethanol is used.

The hydrolysis of the oxazolinyl compound to the 21-hydroxy-20-keto-delta$^{16}$ steroid can be carried out

in an organic solvent, using an acidic aqueous solution. Suitable organics solvents are for instance diethyl ether, methanol and tetrahydrofuran. Suitable acids are diluted strong acids as hydrogen chloride, sulfuric acid and phosphoric acid. Also acetic acid and formic acid can be used.

It is observed that sometimes not only the oxazolinyl group is hydrolyzed but also other groups linked to the steroid skeleton. These groups may have had the function of protective groups (compare what is stated on protective groups in the simultaneously filed application entitled "17-(isocyano-sulfonyl-methylene)-steroids and 17-(formamido-sulfonyl-methylene)-steroids and their preparation").

The invention is elucidated by means of the following examples in which THF = tetrahydrofuran and (Triton B * ) = benzyltrimethylammonium hydroxide (40% solution in methanol).

Example 1a

Preparation of 3-methoxy-17-(2-methoxy-3-oxazolin-4-yl)androsta-3,5,16-triene.

3-Methoxy-17-(isocyano-p-methylphenylsulfonyl-methylene)androsta-3,5-diene (239 mg, 0.5 mmol) was dissolved in 10 ml benzene. To this solution were added formaline (0.2 ml, 40% solution in water), methanol (0.2 ml), benzyl triethyl ammoniumchloride (15 mg) and 50% aqueous NaOH solution (4 ml). The mixture was stirred vigorously for 1 hour at 20 ° C. Extraction with benzene, filtration over $Al_2O_3$ (act. II-III; layer of 2 cm) followed by a washing with methylenechloride (50 ml) and evaporation in vacuo afforded the methoxy-oxazoline (170 mg, 90%). IR (neat): 1655, 1630 $cm^{-1}$. $^1H$ NMR ($CDCl_3$): delta 0.8-2.7 (m), 1.0 (s, 6H), 3.27 (s, 3H), 3.49 (s, 3H), 4.62 (m, 2H), 5.08, 5.18 (m, 2H), 6.2 (m, 1H), 6.45 (m, 1H).
The experiment was repeated, however, in stead of benzyl triethyl ammoniumchloride tetrabutylammonium tetrafluoroborate, tetraethylammonium hydroxide, 18-crown-6 and benzyltriethylammonium hydroxide were used. Yields respectively 93%, 83%, 53% and 91%.

Example 1b

Preparation of 21-hydroxypregna-4,16-diene-3,20-dione.

The above prepared methoxy-oxazoline (170 mg, 0.45 mmole) was dissolved in a mixture of 8% $H_2SO_4$ (2.5 ml) and 10 ml THF. After standing at ambient temperature for 18 hours, water was added (10 ml). The THF was removed by evaporation in vacuo at 20 ° C, whereafter the precipated solid was collected, washed with water and dried. Yield: 149 mg (90%; calculated on the isocyanide) m.p. 215-220 ° C. (dec; acetone petroleum ether 40-60 ° C); $(alpha)^{20}$ +145 ° (c 1.0, $CHCl_3$); IR (Nujol * ) 3350 (OH), 1665 (C=O), 1620 (C=C). $^1H$ NMR ($CDCl_3$): delta 0.8-2.7 (m), 1.0 (s, 3H), 1.2 (s, 3H), 3.68 (s, 1H), 4.4 (s, 2H), 5.65 (s, 1H), 6.68 (m, 1H); Mass: $M^+$ 328 (calcd. 328); (Litt.: Allen and Bernstein, J. Amer. Chem. Soc. 77, 1028 (1975), m.p. 227-232 ° C).

Example 1c

Preparation of 3-methoxy-17-(2-methoxy-3-oxazolin-4-yl)androsta-3,5,16-triene

To a solution of 3-methoxy-17-(isocyano-p-methylphenylsulfonyl-methylene)androsta-3,5-diene (240 mg) in THF under nitrogen formaline (0.2 ml) and Triton B (0.6 ml) were added. After stirring for 15 minutes water was added and the mixture was extracted with methylene chloride. After drying over a layer of alumina and evaporation in vacuo the title compound was obtained (139 mg, 83%). The compound was identical to the one described in Example 1a. The experiment was repeated, however, instead of Triton B tetraethylammonium hydroxide (0.6 ml in water) and methanol (1 ml) were used. Yield: 92%. The experiment was repeated, however, KOH (powdered, 2 g) was used as base. Tetraybutylammonium tetrafluoroborate was used a a catalyst. Yield: 92%. The experiment was repeated, however, benzene (10 ml) was used as solvent, KOH (powdered, 0.65 g) was used as a base and methanol (0.2 ml) was used as alcohol. Yield (after 1 hour stirring): 90%. When in this experiment ethanol is used instead of methanol, the same result is obtained.

* Trade Mark

* Trade Mark

Example 1d

Preparation of 21-hydroxypregna-4,16-diene-3,20-dione

3-Methoxy-17-(isocyano-p-methylphenylsulfonyl-methylene)androsta-3,5-diene (239 mg) is dissolved in 10 ml THF under nitrogen. Formaline (0.2 ml, 36%), methanol (0.2 ml) and powdered KOH (0.8 g) is added. After stirring for 15 minutes the solution is filtered. Sulfuric acid (1.5 ml, 4N) is added followed by stirring for 20 hours. Water is added (10 ml), and the THF is evaporated in vacuo. The residue is filtered and the white solid is dried. Yield: 123 mg (75%) of the title compound.
The same reaction was repeated, however, instead of methanol (0.2 ml) ethanol (0.2 ml) was used. Yield: 146 mg (89%).

Example 2

Preparation of 3-methoxy-21-hydroxy-19-nor-pregna-1,3,5(10),16-tetraen-20-one.

To a solution of 3-methoxy-17-(isocyano-p-methylphenylsulfonyl-methylene)estra-1,3,5(10)-triene (461 mg, 1 mmol) in benzene (20 ml) were added paraformaldehyde (300 mg, 10.0 mmol), methanol (0.4 ml), benzyltriethylammonium chloride (23 mg, 0.1 mmol) and 50% aqueous NaOH solution (8 ml). The mixture was stirred vigorously for one hour, then the organic solvent phase was separated from the water phase and the latter was once extracted with 5 ml of benzene. The combined benzene fractions were filtered over $Al_2O_3$ (act. II-III) followed by a washing with methylene chloride (100 ml). After evaporation of the solvent 3-methoxy-17-(2-methoxy-3-oxazolin-4-yl)-19-nor-pregna-1,3,5(10),16-tetraene was obtained as an oil. Without further processing this oxazolinyl compound was hydrolyzed by dissolving it in 15 ml THF and 5 ml 8% $H_2SO_4$. After 18 hours storage at room temperature the THF was evaporated in vacuum at 20°C. The remaining solid substance was collected, washed with water and dried. The yield was 300 mg (92%), m.p. 120-142°C.
After two recrystallizations from ether/petroleum ether 40-60°C (1:4) the melting point of the product was 144-146°C. (alpha)$^{20}$: + 81° (c 1.00, $CHCl_3$). IR (Nujol * ): 3550 (OH), 1670 (C=O), 1620 (C=C), 1590 (Ar) $cm^{-1}$. $^1H$ NMR ($CDCl_3$): delta 0.9-3.6 (m), 0.95 (s), 3.71 (s, 3H), 4.42 (s, 2H), 6.53, 6.65, 6.71, 7.02, 7.12 (m, 4H). Anal. calcd. for $C_{21}H_{26}O_3$ (326.44): C 77.27, H 8.03; found: C 77.1, H 8.1.

Example 3

Preparation of 21-hydroxypregna-1,4,16-triene-3,20-dione.

17-(isocyano-p-methylphenylsulfonyl-methylene)androsta-1,4-dien-3-one (461 mg, 1.0 mmol) was converted to the hydroxyacetyl compound as described in example 2 (with the difference that during the first reaction step the benzene solution was stirred during half an hour). Yield: 83%, m.p. 155-168°C. After two crystallizations from ether the melting point of the product was 162-174°C. (alpha)$^{20}$: + 92.5° (c 1.00, $CHCl_3$). IR (Nujol * ): 3500 (OH), 1665 (C=O), 1630, 1610, 1590 (C=C) $cm^{-1}$. $^1H$ NMR ($CDCl_3$): delta 0.8-2.7 (m), 1.00 (s), 1.25 (s), 3.25 (br., 1H), 4.40 (s, 2H), 5.95-6.10 (m, 2H), 6.21, 6.23 (d, 1H), 6.6-6.7 (m, 1H). Anal. calcd. for $C_{21}H_{26}O_3$ (326.439): C 77.27, H 8.03; found C 76.7, H 8.1%.

The experiment was repeated under the same conditions, except the stirring time of the phase transfer reaction (first reaction step). Stirring for one hour resulted in a yield of 70%, stirring for two hours resulted in a yield of 52%.

Example 4

Preparation of 21-hydroxypregna-4,9(11),16-triene-3,20-dione.

3-methoxy-17-(isocyano-p-methylphenylsulfonyl-methylene)androsta-3,5,9(11)-triene (475 mg, 1.0 mmol) was converted to the hydroxyacetyl compound as described in example 2. Yield 300 mg, 93%, m.p.

* Trade Mark

* Trade Mark

180-193° C. After two crystallizations from methylene chloride/ether (2:7) the melting point of the product was 205-208° C., (alpha)$^{20}$: + 204° (c 1.00, $CHCl_3$). IR (Nujol * ): 3510 (OH), 1675 (C=O), 1620, 1595 (C=C), 1100 $cm^{-1}$. $^1H$ NMR ($CDCl_3$): delta 0.7-3.3 (m), 0.90 (s), 1.37 (s), 3.06 (s), 4.38 (s, 2H), 5.30-5.60 (m, 2H), 5.27 (s), 6.55-5.80 (m, 1H). (Litt.: J. Amer. Chem. Soc. 77, 1028 (1955); m.p. 204-209° C and m.p. 215-218° C, (alpha)$^{20}$ + 194° (c 1.0, $CHCl_3$)).

Example 5

Preparation of 11-beta,21-dihydroxypregna-4,16-diene-3,20-dione.

3-Methoxy-17-(isocyano-p-methylphenylsulfonyl-methylene)-11-beta-hydroxyandrosta-3,5-diene (493 mg, 1.0 mmol) was converted to the hydroxyacetyl compound as described in example 2 (with the difference that the stirring time during the first reaction step was 3/4 hour). Crude product 233 mg (68%). After crystallization from acetone-ether (1:3) m.p. 148°-153° C, (alpha)$^{20}$: + 198° (c 1.0, $CHCl_3$). IR (Nujol): 3500 (OH), 1690 (C=O), 1655 (br, C=C+C=O) $cm^{-1}$. 1H NMR ($CDCl_3$): delta 0.75-2.9 (m), 1.25 (s), 1.46 (s), 3.0-3.7 (m, 2H), 4.37 (br.s, 3H), 5.57 (s, 1H), 6.48-6.72 (m, 1H).
Litt. J. Amer. Chem. Soc. 77, 1028 (1955); m.p. 154-156° C, (alpha)$^{20}$: + 200° (c 0.47, $CHCl_3$).

It is observed that the yield can be improved by a more thorough washing with $CH_2Cl_2$. $^1H$ NMR of the intermediate 3-methoxy-11-beta-hydroxy-17-(2-methoxy-3-oxazolin-4-yl)androsta-3,5,16-triene: ($CDCl_3$): delta 0.7-2.9 (m), 1.23 (s, 3H), 1.28 (s, 3H), 3.27 (s, 3H), 3.48 (s, 3H), 4.25 (s, 1H), 4.61 (m, 2H), 5.00 (m, 2H), 6.13 (m, 1H), 6.40 (m, 1H).

Example 6

Preparation of 9-alpha-fluoro-11-beta,21-dihydroxypregna-4,16-diene-3,20-dione.

3-Methoxy-9-alpha-fluoro-11-beta-hydroxy-17-(isocyano-p-methylphenylsulfonyl-methylene)androsta-3,5-diene was converted to the hydroxyacetyl compound as described in example 2. Yield 71%, m.p. after crystallization from $CH_2Cl_2$-petroleum ether 40-60° C (1:1) 175-186° C, (alpha)$^{20}$: +165° (c 0.9, $CHCl_3$). IR (Nujol): 3500 (OH), 1670 $cm^{-1}$ (br, C=O). $^1H$ NMR ($CDCl_3$): delta 0.7-3.7 (m), 1.23 (s), 1.57 (s), 4.17 (br.s, 1H), 4.40 (s, 2H), 5.70 (s, 1H), 6.55-6.80 (m, 1H). Litt. US 2,963,496 discloses this compound without giving physical constants.

Example 7

Preparation of 21-hydroxypregna-4,16-diene-3,11,20-trione.

3-Methoxy-17-(isocyano-p-methylphenylsulfonyl-methylene)androsta-3,5-dien-11-one (491 mg, 1.0 mmol) was converted to the hydroxyacetyl compound as described in example 2 with the difference that the stirring time in the first reaction step was 15 minutes. Yield 250 mg (73%), m.p. after crystallization from $CH_2Cl_2$/ether 213-218° C, (alpha)$^{20}$: + 235° (c 1.00, $CHCl_3$). IR (Nujol * ): 3420 (OH), 1710, 1675 (C=O), 1625 (C=C) $cm^{-1}$. $^1H$ NMR ($CDCl_3$): delta 0.8-3.5 (m), 0.91 (s), 1.40 (s), 4.41, 4.48 (d, 2H), 5.68 (s, 1H), 6.64-6.83 (m, 1H). Litt. J. Amer. Chem. Soc. 77, 1028 (1955), m.p. 223-228° C, (alpha)$^{24}$: + 236° (c 1, $CHCl_3$).

Example 8a

Preparation of 1-alpha,2-alpha-methylene-6-chloro-17-(2-methoxy-3-oxazolin-4-yl)androsta-4,6,16-trien-3-one

1-Alpha,2-alpha-methylene-6-chloro-17-(isocyano-p-methylphenylsulfonyl-methylene)androsta-4,6-dien-3-one (218 mg, 0.43 mmol) was dissolved in 8.6 ml benzene. 0.17 ml 40% formaline, 0.17 ml methanol, 0.03 ml Triton B and 3.44 ml aqueous NaOH solution (50%) were successively added to the benzene solution. The mixture was stirred at room temperature for half an hour. Then the two layers were separated

* Trade Mark

* Trade Mark

and the water layer was washed two times with 5 ml benzene. The combined benzene layers were filtered over $Al_2O_3$, followed by a washing with methylene chloride. After a further purification step by means of a chromatography with toluene, as well as increasing quantities of acetone, 94 mg of the methoxy-oxazolinyl compound was obtained, m.p. 164-167 °C. IR 1655 (C(3) = O), 1630 (C = N), 1060 (COC) $cm^{-1}$. $^1H$ NMR ($CDCl_3$): delta 0.7-1.0 (m, cyclopropyl), 1.096 (s, 3H), 1.261 (s, 3H), 3.31 (s, 3H), 4.65 (m, 2H), 6.15 (m, 2H), 6.22 (m, 1H).

Example 8b

Preparation of 1-alpha,2-alpha-methylene-6-chloro-21-hydroxypregna-4,6,16-triene-3,20-dione.

The methoxy-oxazolinyl compound prepared according to example 8a (90 mg) was dissolved in 8 ml THF, then 3 ml 2N $H_2SO_4$ was added. The mixture was stored at room temperature for 16 hours. After addition of $NaHCO_3$ (540 mg, 6 mmol) and some water the THF was removed by evaporation. The remaining solid substance was collected, washed with water and dried. Thus 57 mg of the hydroxyacetyl compound was obtained, m.p. 175-178 °C. IR ($CHCl_3$): 1648, 1665 ($C^3$ = O and $C^{20}$ = O), 1588, 1608 (C = C) $cm^{-1}$. $^1H$ NMR ($CDCl_3$): delta 1.030 (s, 3H), 1.258 (s, 3H), 3.1 (s, br, 1H), 4.47 (s, 2H), 6.18 (m, 2H), 6.77 (m, 1H).

Example 9a

Preparation of 3,3-ethyleendithio-17-(2-methoxy-3-oxazolin-4-yl)-androsta-4,16-diene.

3,3-Ethylenedithio-17-(isocyano-p-methylphenylsulfonyl-methylene)androsta-4-ene (540 mg, 1 mmol) was dissolved in 20 ml benzene. Then 0.4 ml formaline (40%), 0.4 ml methanol, 0.4 ml triton B and 8 ml 50 % aqueous NaOH solution were successively added. The mixture was stirred at room temperature during 20 minutes. After dilution with water the benzene layer was separated. The aqueous layer was extracted one time with 10 ml benzene. The collected benzene solutions were filtered over $Al_2O_3$ (act. II-III) and washed with $CH_2Cl_2$. After evaporation of the solvent 0.5 g of the oxazolinyl compound was obtained. $^1H$ NMR ($CDCl_3$): delta 1.01 (s, 3H), 1.07 (s, 3H), 3.33 (m, 7H), 4.3-4.9 (m, 2H), 5.53 (s, 1H), 6.30 (s, 1H), 6.59 (t, 1H).

Example 9b

Preparation of 3,3-ethylenedithio-21-hydroxypregna-4,16-dien-20-one.

The oxazolinyl compound prepared according to example 9a (500 mg) was dissolved in 10 ml THF and 2.5 ml $H_2SO_4$ (8%).The mixture was stored at room temperature for 22 hours, then water (10 ml) was added and the THF removed by evaporation under reduced pressure. 280 mg of the hydroxyacetyl compound was obtained, m.p. 206-210 °C (browning).
IR ($CHCl_3$): 3475 (OH), 1665 (C = O), 1586 ($delta^{16}$) $cm^{-1}$. $^1H$ NHR ($CDCl_3$): delta 0.95 (s, 3H), 1.06 (s, 3H), 3.32 (s, 4H), 4.46 (s, 2H), 5.50 (s, 1H), 6.76 (t, 1H).

Example 10a

Preparation of 3,3-ethylenedioxy-17-(2-methoxy-3-oxazolin-4-yl)-androsta-5,16-diene.

3,3-Ethylenedioxy-17-(isocyano-p-methylphenylsulfonyl-methylene)androsta-5-ene (507 mg, 1 mmol) was dissolved 20 ml benzene. Then 0.4 ml formaline (40%), 0.4 ml methanol, 0.06 ml Triton B and 8 ml 50 % aqueous NaOH solution were added. The reaction mixture was stirred for half an hour at room temperature. Then the water layer was separated and washed two times with benzene. The combined benzene layers were filtered over $Al_2O_3$ (act. II-III), layer thickness 3.5 cm, and washed with $CH_2Cl_2$. The solvent was evaporated and thus 296 mg (72%) of the oxazolinyl compound, a white solid, was obtained: m.p. 154-157 °C. IR ($CHCl_3$): 1623 (C = N), 1093 (C-O-C) $cm^{-1}$. $^1H$ NMR ($CDCl_3$): delta 1.017 (s, 3H), 1.077 (s, 3H), 3.29, 3.39 (2xs, 6H), 3.92 (s, 4H), 4.66 (m, 2H), 5.35 (m, 1H), 6.27 (m, 1H), 6.54 (t, 1H).

Example 10b

Preparation of 21-hydroxypregna-4,16-diene-3,20-dione.

The oxazolinyl compound prepared according to example 10a (222 mg) was dissolved in 15 ml THF, then 4.5 ml 2 N $H_2SO_4$ were added. The solution was kept for 16 hours at room temperature. The THF was evaporated and the remaining solid was washed with water. After drying under reduced pressure 178 mg substance were obtained. However, hydrolysis was not yet complete. Therefore a further reaction with 4 N $H_2SO_4$ in THF was applied, resulting into the pure hydroxyacetyl compound (162 mg, 92%). IR and NMR were identically with the spectra of the product obtained in example 1b.

Example 11a

Preparation of 3-beta-(2'-tetrahydropyranyloxy)-17-(2-methoxy-3-oxazolin-4-yl-)androsta-5,16-diene.

3-Beta(2'-tetrahydropyranyloxy)-17-(isocyano-p-methylphenylsulfonyl-methylene)androsta-5-ene (570 mg, 1.04 mmol) was dissolved in 21 ml benzene, then formaline (0.42 ml), methanol (0.42 ml), Triton B (0.062 ml) and an aqueous NaOH solution (50%; 8.3 ml) were added. The reaction mixture was stirred at room temperature for 25 minutes. The benzene layer was separated and the water layer was washed with two portions of 5 ml benzene. The combined benzene solutions were filtered over $Al_2O_3$ (act. II-III) and washed with 100 ml $CH_2Cl_2$. After evaporation of the solvent the oxazolinyl compound was obtained (330 mg). IR ($CHCl_3$): 1623 (C=N), 1055, 1025 (C-O-C). $^1$H NMR ($CDCl_3$): delta 1.017 (s, 3H), 5.30 (m, 1H), 6.22 (m, 1H), 6.48 (m, 1H).

Example 11b

Preparation of 3-beta,21-dihydroxypregna-5,16-dien-20-one.

The oxazolinyl compound prepared according to example 11a (152 mg, 0.27 mmol) was dissolved in 12 ml THF, then 3.5 ml 4 N $H_2SO_4$ was added. After storage at room temperature for 20 hours $NaHCO_3$ (1.359 g, 16 mmol) and 25 ml water were added. The THF was evaporated, the remaining solid collected, washed with water and dried. Column-purification using toluene-acetone (9:1) afforded 64 mg of the pure hydroxyacetyl compound; m.p. 194-197 °C (browning at 187 °C). IR ($CHCl_3$): 3610, 3470 (OH), 1666 ($C^{20}$=O), 1585 (C=C) $cm^{-1}$. $^1$H NMR ($CDCl_3$): delta 0.96 (s, 3H), 1.03 (s, 3H), 3.18 (s, 1H), 3.35 (m, 1H), 4.33 (m, 2H), 5.24 (m, 1H), 6.74 ($C^{16}$H).

Example 12a.

Preparation of 3-methoxy-11-alpha-hydroxy-17-(2-methoxy-3-oxazolin-4-yl)androsta-3,5,16-triene.

To a solution of 3-methoxy-11-alpha-hydroxy-17-(isocyano-p-methylphenylsulfonyl-methylene)androsta-3,5-diene (496 mg, 1 mmol) in toluene (20 ml) were added paraformaldehyde (300 mg, 10 mmol), methanol (0.8 ml), benzyltriethyl ammonium chloride (25 mg) and 50 % aqueous NaOH solution (8 ml). The mixture was stirred vigorously for 25 minutes. Extraction with toluene, filtration over $Al_2O_3$ (act. II-III), followed by a washing with methylene chloride and evaporation in vacuo afforded the oxazoline (196 mg). IR ($CHCl_3$): 1653, 1623 (C=C, C=N) $cm^{-1}$. $^1$ H NMR ($CDCl_3$): delta 1.048 (s, 3H), 1.174 (s, 3H), 3.35 (s, 3H), 3.57 (s, 3H), 4.20 (m, 1H), 4.70 (m, 2H), 5.14 (s, 1H), 5.76 (m, 1H), 6.32 (tr, 1H), 6.57 (m, 1H).

Example 12 b

Preparation of 11-alpha,21-dihydroxypregna-4,16-diene-3,20-dione.

The oxazolinyl compound prepared according to example 12a (98 mg) was dissolved in 5 ml THF, then 1.25 ml 4 N $H_2SO_4$ was added. After storage at room temperature for 20 hours 5 ml water was added. The THF was evaporated and the remaining substance was collected. Yield: 69 mg. IR ($CHCl_3$): 3598 (OH), 3470 (OH), 1660 (C=O), 1615, 1589 (C=C) $cm^{-1}$. $^1$H NMR ($CDCl_3$) delta 0.996 (s, 3H), 1.271 (s, 3H), 4.20 (m, 1H), 4.48 (s, 2H), 5.77 (s. 1H), 6.80 (tr, 1H).

Example 13 a

Preparation of 3-methoxy-6-chloro-17-(2-methoxy-3-oxazolin-4-yl)androsta-3,5,16-triene.

To a solution of 1 g 3-methoxy-6-chloro-17-(isocyano-p-methylphenylsulfonyl-methylene)androsta-3,5-diene in 50 ml toluene were added 1 ml methanol, 1 ml formaline (40%), 75 mg benzyltriethyl ammonium chloride and 20 ml aqueous NaOH solution (50%).The mixture was stirred vigorously for 1.5 hour at ambient temperature. Extraction with toluene, filtration over $Al_2O_3$ (act. II-III) followed by a washing with methylene chloride and evaporation afforded the oxazoline. Yield: 0.49 g. IR ($CHCl_3$): 1643, 1619 (C=C + N=C) $cm^{-1}$. $^1$H NMR ($CDCl_3$): delta 1.047 (s, 6H), 3.36 (s, 3H), 3.65 (s, 3H), 4.71 (m, 2H), 5.67 (s, 1H), 6.32 (tr, 1H), 6.58 (tr, 1H).

Example 13b

Preparation of 3-methoxy-6-chloro-21-hydroxypregna-3,5,16-trien-20-one.

The oxazolinyl compound prepared according to example 13a (400 mg) was dissolved in 20 ml THF, then 5 ml THF was added. After storage at ambient temperature for 18 hours 20 ml water was added. The THF was evaporated and the resulting substance was extracted with methylene chloride. The organic layer was dried and concentrated. Colomn-purification using toluene afforded 120 mg of the pure hydroxyacetyl compound; m.p. 158-160°C. IR ($CHCl_3$): 3471 (OH), 1670 (C=O), 1645, 1620, 1588 (C=C) $cm^{-1}$. NMR ($CDCl_3$): delta 0.983 (s, 3H), 1.044 (s, 3H), 3.65 (s, 3H), 4.48 (s, 2H), 5.66 (s, 1H), 6.78 (tr, 1H).

Example 14a

Preparation of 3-beta-methoxymethoxy-17-(2-methoxy-3-oxazolin-4-yl)androsta-5,16-diene.

3-Beta-methoxymethoxy-17-(isocyano-p-methylphenylsulfonyl-methylene)androst-5-ene (509 mg, 1 mmol) was converted to the oxazolinyl compound as described in example 13a (with the difference that the mixture was h stirred for 35 minutes). Yield 193 mg. $^1$H NMR ($CDCl_3$): delta 0.99 (s, 3H), 1.04 (s, 3H), 3.33 (s, 7H), 4.65 (m, 4H), 5.35 (m, 1H), 6.35 (m, 1H), 6.53 (m, 1H).

Example 14b

Preparation of 3-beta-methoxymethoxy-21-hydroxypregna-5,16-dien-20-one.

The oxazolinyl compound prepared according to example 14a (103 mg) was dissolved in 6 ml THF, then 1.5 ml 2N $H_2SO_4$ was added. After storage at room temperature for 48 hurs 15 ml water was added. The THF was removed by evaporation in vacuo, whereafter the precipitated solid was collected, washed with water and dried. Yield: 37 mg. $^1$H NMR ($CDCl_3$): delta 0.95 (s, 3H), 1.05 (s, 3H), 3.34 (s, 4H), 4.42 (s, 2H), 4.64 (s, 2H), 5.35 (m, 1H), 6.71 (m, 1H).

Example 15a

Preparation of 17-(2-methoxy-3-oxazolin-4-yl)androsta-4,16-dien-3-one

17-(isocyano-p-methylphenylsulfonyl-methylene)androst-4-en-3-one (200 mg) was converted to the methoxy-oxazolinyl compound as described in example 1a (with the difference that the mixture was stirred for 20 minutes). Yield 120 mg. $^1$H NMR ($CDCl_3$): delta 0.99 (s, 3H), 1.18 (s, 3H), 3.28 (s, 3H), 4.64 (m, 2H), 5.68 (s, 1H), 6.23 (m, 1H), 6.49 (m, 1H).

Example 15b

Preparation of 21-hydroxypregna-4,16-diene-3,20-dione

The oxazolinyl compound prepared according to example 15a (120 mg) was converted to the hydroxyacetyl compound as described in example 14b. Yield: 37 mg. IR and NMR were identically with the spectra of the products obtained in examples 1b and 10b.

Example 16

Preparation of 21-hydroxy-19-nor-pregna-4,16-diene-3,20-dione

To a solution of 351 mg 3-methoxy-17-(isocyano-p-methylphenylsulfonyl-methylene)-19-nor-androsta-3,5-diene in 15 ml toluene were added 0.3 ml formaline (40%), 0.3 ml methanol, 22 mg benzyltriethyl ammonium chloride and 6 ml aqueous NaOH solution (50%). The mixture was stirred vigorously for 65 minutes at ambient temperature. Extraction with toluene, filtration over $Al_2O_3$ (act. II-III) followed by a washing with methylene chloride and evaporation in vacuo afforded 112 mg 3-methoxy-17-(2-methoxy-3-oxazolin-4-yl)-19-nor-androsta-3,5,16-triene. Without further processing this oxazolinyl compound was hydrolyzed by dissolving it in 8 ml THF and 2 ml $H_2SO_4$.After stirring for 17 houres at room temperature, 20 ml water was added. The THF was removed by evaporation in vacuo, whereafter the precipitated solid was collected, washed with water and dried. Yield: 58 mg. IR ($CHCl_3$): 3475 (OH), 1666 (C = O), 1619, 1588 (C = C) $cm^{-1}$. $^1H$ NMR ($CDCl_3$): delta 1.02 (s, 3H), 3.24 (s, 1H), 4.48 (s, 2H), 5.87 (s, 1H), 6.78 (tr, 1H).

Example 17

Preparation of 1-alpha-methyl-3-methoxy-17-(2-methoxy-3-oxazolin-4-yl)androsta-3,5,16-triene.

The oxazolinyl compound was prepared as described in example 9a, starting from 1-alpha-methyl-3-methoxy-17-(isocyano-p-methylphenylsulfonyl-methylene)-androsta-3,5-diene (982 mg, 2 mmol). Yield: 653 mg (83 %). IR ($CHCl_3$): 1628 (C = N), 1057 (COC). $^1H$ NMR ($CDCl_3$): delta 0.78 (d, 3H), 1.03 (s, 3H), 1.07 (s, 3H), 3.30 (s, 3H), 3.52 (s, 3H), 4.65 (m, 2H), 5.06 (m, 1H), 5.29 (m, 1H), 6.25 (m, 1H), 6.52 (m, 1H). In the same way as described in Example 9b the title compound can be hydrolyzed to 1-alpha-methyl-21-hydroxypregna-4,16-diene-3,20-dione.

Example 18

Preparation of 3-methoxy-17-(2-methoxy-3-oxazolin-4-yl)androsta-3,5,16-triene

According to the method described in Example 2 the title compound was prepared from a number of 3-methoxy-17-(isocyano-sulfonyl-methylene)androsta-3,5-diene compounds with different groups attached to the sulfonyl group. The following results were obtained.

| | | | |
|---|---|---|---|
| R = | $-C_6H_4CH_3$ | Yield = | 92% |
| | $-C_6H_4-OCH_3$ | | 82% |
| | $-C_6H_4-p-Cl$ | | 97% |
| | $-n-(CH_2)_9CH_3$ | | 86% |
| | $-t-C_4H_9$ | | 6% |
| | $-CH_3$ | | 90% |

Example 19

Preparation of 3-methoxy-17-(2-allyloxy-3-oxazolin-4-yl)androsta-3,5,16-triene

The title compound was prepared according to the method described in Example 2, starting from 477 mg of the starting compound of Example 1 and using 0.5 ml allyl alcohol (7 mmol) instead of methanol. Yield: 350 mg (86%). IR (Nujol * ): 1655, 1630. $^1H$ NMR ($CDCl_3$): delta 0.8-2.8 (m), 1.0 (s, 6H), 3.5 (s, 3H), 3.98, 4.18 (d, 2H), 4.5-4.7 (m, 2H), 3.9-4.4 (m, 4H), 4.5-6.05 (m, 1H), 6.05-6.3 (m, 1H), 6.3- 6 (m, 1H). 3-Methoxy-17-(2-benzyloxy-3-oxazolin-4-yl)androsta-3,5,16-triene was prepared in the same way, however, using benzyl alcohol (1 ml, 10 mmol) instead of allyl alcohol. Yield: 52%, m.p. 115-125 °C. IR (Nujol * ): 1630 (N = C). $^1H$ NMR ($CDCl_3$): delta 0.8-2.8 (m), 1.06 (s), 3.58 (s, 3H), 4.55-4.9 (m, 4H), 4.65 (s), 5.1-5.4 (m, 2H), 6.15-6.45 (m, 1H), 7.34 (s, 5H). Exact mass 459.276 (calcd. 459.277).

* Trade Mark

Example 20

Preparation of 3-methoxy-17-(2-methoxymethoxy-3-oxazolin-4-yl)androsta-3,5,16-triene

3-Methoxy-17-(isocyano-p-methylphenylsulfonyl-methylene)androsta-3,5-diene (239 mg, 0.5 mmol) was dissolved in benzene (7.5 ml). Sodium hydroxide (2.5 mmol, 100 mg), 15-crown-5 (0.10 ml, 0.5 mmol), paraformaldehyde (150 mg, 5 mmol) and methanol (0.2 ml) were added. After stirring for 3.5 hours the title compound was isolated from the reaction mixture in the usual way. Yield: 96%. IR (Nujol): 1625 $cm^{-1}$. $^1H$ NMR ($CDCl_3$): delta 0.9-2.8 (m), 1.82 (s, 3H), 3.42 (s, 3H), 3.55 (s, 3H), 4.6-5.05 (m, 4H), 5.1-5.4 (m, 2H), 6.2-6.4 (m, 1H), 6.6-6.9 (m, 1H). M.p. 145-165 °C ($CH_2Cl_2$/MeOH). Anal. calcd. for $C_{25}H_{35}NO_4$ (413.563): C 72.61, H 8.53, N 3.39. Found: C 72.3, H 8.7, N 3.4. Remark: methoxymethanol is probably formed by reaction of a molecule formaldehyde and a molecule methanol.

Example 21a

Preparation of 3-isobutoxy-17-(2-methoxy-3-oxazolin-4-yl)androsta-3,5,16-triene

3-Isobutoxy-17-(isocyano-p-methylphenylsulfonyl-methylene)androsta-3,5-diene (520 mg, 1 mmol) was converted to the methoxy-oxazolinyl compound as described in example 13a with the difference that tetrabutylammonium tetrafluoroborate was used as phase transfer catalyst. Yield: 240 mg. $^1H$ NMR ($CDCl_3$): delta 0.95, (d, 6H), 1.046 (s, 6H), 3.34 (s, 3H), 3.47 (d, 2H), 4.70 (m, 2H), 5.12 (s, 1H), 5.18 (tr, 1H), 6.30 (tr, 1H), 6.57 (tr, 1H) ppm.

Example 21b

Preparation or 21-hydroxypregna-4,16-diene-3,20-dione

The oxazolinyl compound prepared according to example 21a (192 mg) was converted to the hydroxyacetyl compound as described in example 1b. Yield: 119 mg (73%). IR was identically with the IR of the product obtained in example 1b.

Example 22a

Preparation of 3-methoxy-9-alpha-hydroxy-17-(2-methoxy-3-oxazolin-4-yl)androsta-3,5,16-triene

3-Methoxy-9-alpha-hydroxy-17-(isocyano-p-methylphenylsulfonyl-methylene)androsta-3,5-diene (494 mg, 1 mmol) was converted to the methoxy-oxazolinyl compound as described in example 13a. Yield: 170 mg. $^1H$ NMR ($CDCl_3$): delta 1.045 (s, 3H), 1.146 (s, 3H), 3.36 (s, 3H), 3.58 (s, 3H), 4.70 (m, 2H), 5.17 (s, 1H), 5.31 (tr, 1H), 6.30 (tr, 1H), 6.57 (d, 1H) ppm.

Example 22b

Preparation of 9-alpha,21-dihydroxypregna-4,16-diene-3,20-dione.

The oxazolinyl compound prepared according to example 22a (97 mg) was dissolved in 6 ml THF and 1.5 ml 2N $H_2SO_4$. The mixture was stored at room temperature for 22 hours, then water (10 ml) was added and the THF removed by evaporation under reduced pressure. 34 mg of the hydroxyacetyl compound was obtained. IR (KBr): 3400 (OH), 1664 (C=O, 2x), 1612, 1579 (C=C). $^1H$ NMR ($CDCl_3$): 0.992 (s, 3H), 1.369 (s, 3H), 2.95 (br, s, OH), 3.58 (br, s, OH), 4.40-4.54 (AB, 2H), 5.81 (s, 1H), 6.81 (tr, 1H).

Example 23

Preparation of 21-hydroxy-pregna-4,16-diene-3,20-dione

Potassium-t-butoxide (840 mg, 7.5 mmol) was added to dry tetrahydrofuran (25 ml) whereafter the suspension was cooled to -70 °C. TosMIC (1.17 g, 6 mmol) dissolved in 10 ml THF was added to the suspension. After 10 minutes stirring at -70 °C 3-methoxyandrosta-3,5-dien-17-one (1.5 g, 5 mmol) dissolved in 15 ml THF was added. The mixture was stirred for two hours at -40 ° - -30 °C, followed by the

addition of phosphoric acid (0.64 g, 7.5 mmol) at -35° C. After stirring for 10 minutes, triethylamine (7.5 ml, 54 mmol) and phosphoroxychloride (1 ml, 11 mmol) were added at -35° C. The reaction mixture was stirred for one hour at 0° C, and formaline (2 ml, 36%), methanol (2 ml) and KOH (powdered, 7 g) were added. After stirring for 20 minutes the reaction mixture was filtered. Sulfuric acid (20 ml, 4N) was added, followed by 10 ml THF. After stirring for 20 hours the THF was evaporated in vacuo. The crystals were sucked dry, washed with water and dried. Yield: 1.22 g (74%) of the title compound, which was identical with the compound made in Example 1b.

## Claims

**1.** Process for the preparation of 21-hydroxy-20-keto-delta$^{16}$-steroids of formula III:

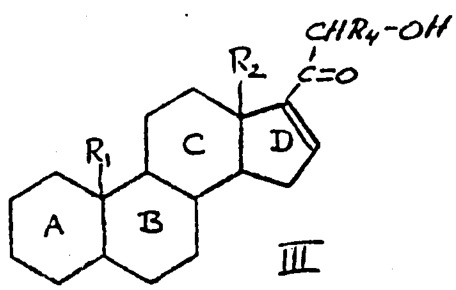

III

in which $R_1$ represents a hydrogen atom or an alkyl group having 1 to 4 carbon atoms, or is absent in the case of a double bond between $C_{10}$ and $C_1$, $C_5$ or $C_9$,
$R_2$ represents a hydrogen atom or an alkyl group having 1 to 4 carbon atoms,
$R_4$ represents a hydrogen atom or an alkyl group having 1 to 4 carbon atoms and optionally substituted by one or more halogen atoms or alkoxy groups or a phenyl group optionally substituted by one or more halogen atoms or alkyl or alkoxy groups
and the rings A, B, C and D may contain one or more double bonds and may be substituted by one or more of the following groups: hydroxy, amino, oxo, halogen, alkyl, alkylene, alkoxy, alkoxyalkoxy, epoxy, methylene, alkylenedioxy, alkylenedithio or alkyleneoxythio characterized by reacting a 17-(isocyano-sulfonyl-methylene) steroid of formula I:

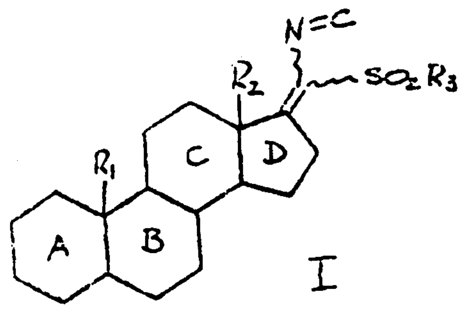

I

in which $R_1$ and $R_2$ have the meaning as listed above and rings A, B, C and D may contain double bonds and carry substituents as defined above and in which $R_3$ represents an alkyl group, an aryl group substituted or not substituted by one or more halogen atoms or alkyl, alkoxy, nitro or cyano groups, or represents a dialkylamino group, wherein the alkyl groups together with the nitrogen atom may form a heterocyclic ring wish up to 8 ring atoms and optionally containing an oxygen atom under basic conditions with an aldehyde $R_4HO$, where
$R_4$ represents a hydrogen atom or an alkyl group having 1 to 4 carbon atoms and optionally substituted by one or more halogen atoms or alkoxy groups or a phenyl group optionally substituted by one or more halogen atoms or alkyl or alkoxy groups and with an alkyl alcohol or an arylalkyl alcohol, and followed by hydrolysis.

**2.** Process for the preparation of 17-(2-$OR_5$-3-oxazolin-4-yl)-delta$^{16}$ steroids of formula II

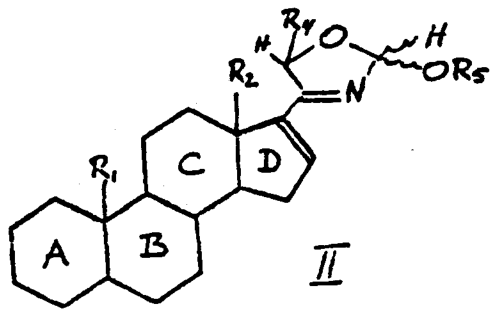

II

in which $R_1$, $R_2$ and $R_4$ have the meanings as defined in claim 1, rings A, B, C and D may contain double bonds and carry substituents as defined in claim 1 and $R_5$ represents an alkyl or arylalkyl group, characterized by reacting a 17-(isocyano-sulfonyl-methylene) steroid according to claim 1 under basic conditions with an aldehyde and an alcohol as defined in claim 1.

**3.** Process according to claim 1 or 2, characterized in that the reaction is carried out in an inert organic solvent using a metal hydroxide, a metal alcoholate or a quaternary ammonium or phosphonium hydroxide as base.

**4.** Process according to claim 3, characterized in that a catalyst is added to the reaction mixture in the form of a quaternary ammonium or phosphonium salt or a crown ether.

**5.** Process according to claim 1 or 2, characterized in that the reaction is carried out under phase-transfer conditions.

**6.** Process according to claims 1-5, characterized in that the aldehyde is a phenylaldehyde in which the phenyl group optionally is substituted by one or more halogen atoms or alkyl or alkoxy groups or an alkyl aldehyde in which the alkyl group has 1 to 4 carbon atoms and optionally is substituted by one or more halogen atoms or alkoxy groups, preferably benzaldehyde or acetaldehyde.

**7.** Process according to claim 1-6, characterized in that the aldehyde is formaldehyde.

**8.** Process according to claims 1-7, characterized in that the alcohol is an alkyl alcohol or arylalkylalcohol, preferably a $C_1$-$C_6$ alkylalcohol.

**9.** Process according to claims 1-8, characterized in that the alcohol is methanol or ethanol.

**10.** Process for the preparation of 21-hydroxy-20-keto-delta$^{16}$-steroids according to claim 1, characterized by hydrolysis of the 17-(2-$OR_5$-3-oxazolin-4-yl)-delta$^{16}$-steroids according to claim 2.

**11.** Process according to claim 1 or 10, characterized in that the hydrolysis is carried out in an organic solvent using an acidic aqueous solution.

**12.** Process according to claims 1-11, characterized in that the 17-(isocyano-sulfonyl-methylene) steroid has the general formula:

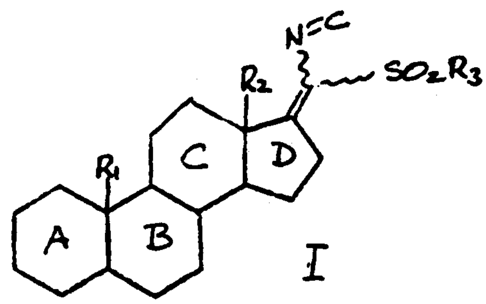

I

or the 17-(2-$OR_5$-3-oxazolin-4-yl)-delta$^{16}$ steroid has the general formula:

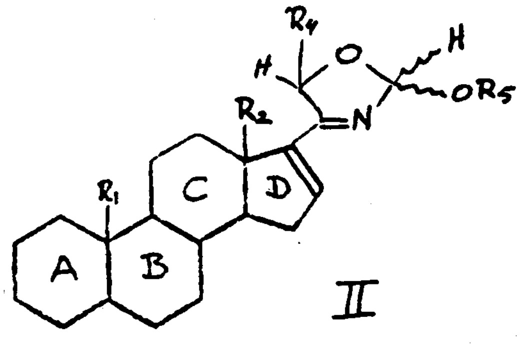

II

in which $R_1$ represents a hydrogen atom or a methyl group or is absent in the case of a double bond between $C_{10}$ and $C_1$, $C_5$ or $C_9$, $R_2$ represents a hydrogen atom or a methyl group, $R_3$ represents an alkyl or dialkylamino group, or an aryl group substituted or not substituted by one or more halogen atoms, alkyl, alkoxy, nitro or cyano groups,
$R_4$ represents a hydrogen atom or an alkyl group having 1 to 4 carbon atoms and optionally substituted by one or more halogen atoms or alkoxy groups or a phenyl group optionally substituted by one or more halogen atoms or alkyl or alkoxy groups,
$R_5$ represents an alkyl or arylakyl, group, and the rings A, B, C and D optionally contain one or more double bonds, and are optionally substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, or alkylenedioxy, alkylenedithio or alkyleneoxythio groups.

**13.** Process according to claims 1-12, characterized in that $R_4$ represents a hydrogen atom and $R_5$ represents a methyl group.

**14.** Process as defined in claim 12 or 13, characterized in that $R_3$ represents an alkyl group having 1 to 10 carbon atoms or $R_3$ represents a phenyl or naphthyl group optionally substituted by a halogen atom, one or more alkyl groups or an alkoxy group, preferably $R_3$ represents a phenyl, p-methoxyphenyl or p-methylphenyl group.

**15.** Process according to claims 12-14 in which the rings A, B, C and D contain one or more double bonds characterized in that these double bonds are present between $C_1$ and $C_2$, $C_3$ and $C_4$, $C_4$ and $C_5$, $C_5$ and $C_6$, $C_6$ and $C_7$, $C_9$ and $C_{11}$ and/or $C_{11}$ and $C_{12}$.

**16.** Process according to claims 12-15, characterized in that the rings A, B, C and D are substituted by one or more 3-, 9-, 11-, 12- or 14-hydroxy groups, and/or by an oxygen atom at $C_3$, $C_{11}$ or $C_{12}$ and/or by one or more 6-, 9- or 11-fluorine, chlorine or bromine atoms, and/or by a 1- or 6-methyl group, and/or by a 3-, 9- or 11-alkoxy group containing 1-4 carbon atoms, and/or by a 3- or 11- alkoxyalkoxy group.

**17.** Process according to claims 12-16, characterized in that the rings A, B, C and D are disubstituted by

an epoxy group at $C_1$ and $C_2$ or $C_9$ and $C_{11}$ and/or by a methylene group attached to $C_1$ and $C_2$ and/or by a 3,3-alkylenedioxy, a 3,3-alkylenedithio or a 3,3-alkyleneoxythio group.

**18.** 17-(2-$OR_5$-3-oxazolin-4-yl)-delta$^{16}$ steroids with the general formula:

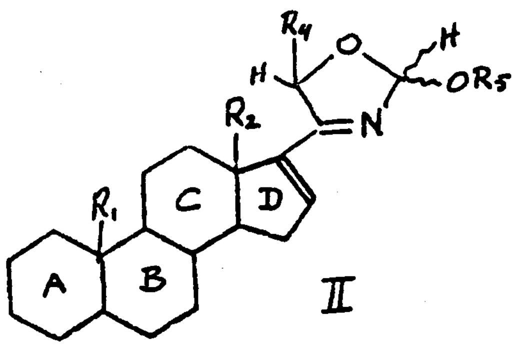

II

in which $R_1$ represents a hydrogen atom or a methyl group or is absent in the case of a double bond between $C_{10}$ and $C_1$, $C_5$, or $C_9$, $R_2$ represents a hydrogen atom or a methyl group, $R_4$ represents a hydrogen atom or an alkyl or a phenyl group, $R_5$ represents an alkyl, group, and the rings A, B,C, and D optionally contain one ore more double bonds, and are optionally substituted by one or more hydroxy groups, amino groups, oxygen atoms, halogen atoms or alkyl, alkylene, alkoxy or alkoxyalkoxy groups and are disubstituted or not disubstituted by one or more epoxy groups methylene groups or alkylenedioxy, alkylenedithio or alkyleneoxythio groups.

## Revendications

**1.** Procédé de préparation de 21-hydroxy-20-céto-delta$^{16}$-stéroïdes de formule III:

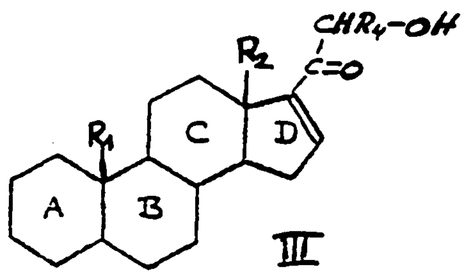

III

dans laquelle $R_1$ représente un atome d'hydrogène ou un radical alkyle comportant 1 à 4 atomes de carbone, ou bien est absent quand une double liaison existe entre $C_{10}$ et $C_1$, $C_5$ ou $C_9$, $R_2$ représente un atome d'hydrogène ou un radical alkyle comportant 1 à 4 atomes de carbone, $R_4$ représente un atome d'hydrogène ou un radical alkyle comportant 1 à 4 atomes de carbone, éventuellement substitué par un ou plusieurs atomes d'halogène ou radicaux alkyloxy ou un radical phényle éventuellement substitué par un ou plusieurs atomes d'halogène ou radicaux alkyle ou alkyloxy, et les noyaux A, B, C et D peuvent contenir une ou plusieurs double liaison et être substitués par un ou plusieurs des radicaux suivants: hydroxy, amino, oxo, halogène, alkyle, alkylène, alkyloxy, alkyloxyalkyloxy, époxy, méthylène, alkylènedioxy, alkylènedithio ou alkylènoxythio, caractérisé en ce que l'in fait réagir dans des conditions basiques, un 17-(isocyano-sulfonyl-méthylène) stéroïde de formule I:

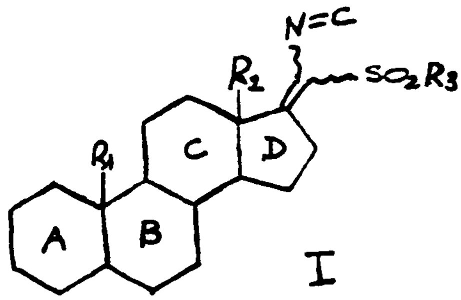

I

dans laquelle $R_1$ et $R_2$ ont la signification indiquée ci-dessus, et les noyaux A, B, C, et D peuvent contenir des doubles liaisons et porter des substituants tels que définis ci-dessus, et dans laquelle $R_3$ représente un radical alkyle ou un radical aryle éventuellement substitué par un ou plusieurs atomes d'halogène ou radicaux alkyle, alkyloxy, nitro ou cyano, ou bien représente un groupe dialkyl-amino dans lequel les radicaux alkyle peuvent former avec l'atome d'azote un noyau hétérocyclique comportant jusqu'à 8 atomes cycliques et contenant éventuellement un atome d'oxygène,
avec un aldéhyde $R_4CHO$ où $R_4$ représente un atome d'hydrogène ou un radical alkyle comportant 1 à 4 atomes de carbone et éventuellement substitué par un ou plusieurs atomes d'halogène ou radicaux alkyloxy ou un radical phényle éventuellement substitué par un ou plusieurs atomes d'halogène ou radicaux alkyle ou alkyloxy,
et un alcanol ou un arylalcanol puis que l'on hydrolyse.

2. Procédé de préparation de 17-(2-$OR_5$ 3-oxazolin-4-yl) delta$^{16}$ -stéroïdes de formule II

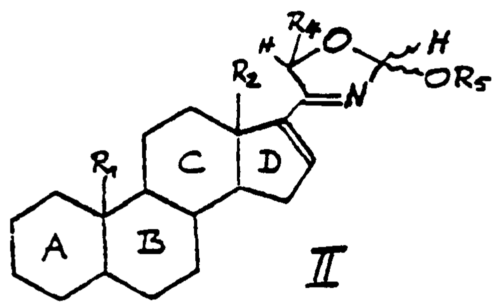

II

dans laquelle $R_1$, $R_2$ et $R_4$ ont la signification indiquée dans la revendication 1, les noyaux A, B, C et D peuvent contenir des doubles liaisons et porter les substituants tels que spécifiés dans la revendication 1, et $R_5$ représente un radical, alkyle ou arylalkyle, caractérisé en ce que l'on fait réagir dans des conditions basiques suivant la revendication 1, un 17-(isocyano-sulfonylméthylène)-stéroïde, avec un aldéhyde et un alcool, tels que définis dans la revendication 1.

3. Procédé selon la revendication 1 ou 2, caractérisé en ce que la réaction est effectuée dans un solvant organique inerte en utilisant comme base un hydroxyde de métal, un alcoolate de métal ou un hydroxyde d'ammonium ou de phosphonium quaternaire.

4. Procédé selon la revendication 3, caractérisé en ce qu'un catalyseur est ajouté au mélange réactionnel sous forme d'un sel quaternaire, soit d'ammonium soit de phosphonium, ou d'un éther-couronne.

5. Procédé selon la revendication 1 ou 2, caractérisé en ce que la réaction est effectuée dans des conditions de transition de phase.

6. Procédé selon les revendications 1 à 5, caractérisé en ce que l'aldéhyde est un phénylaldéhyde dans lequel le radical phényle est éventuellement substitué par un ou plusieurs atomes d'halogène ou radicaux alkyle ou alkyloxy, ou un alkyl-aldéhyde dans lequel le radical alkyle comporte 1 à 4 atomes de carbone et est éventuellement substitué par un ou plusieurs atomes d'halogène ou radicaux alkyloxy, de préférence le benzaldéhyde ou l'acétaldéhyde.

**7.** Procédé selon les revendications 1 à 6, caractérisé en ce que l'aldéhyde est le formaldéhyde.

**8.** Procédé selon les revendications 1 à 7, caractérisé en ce que l'alcool est un alcanol ou un arylalcanol, de préférence un alcanol $C_1$ - $C_6$.

**9.** Procédé selon les revendications 1 à 8, caractérisé en ce que l'alcool est le méthanol ou l'éthanol.

**10.** Procédé de préparation de 21-hydroxy 20-céto delta$^{16}$-stéroïdes selon la revendication 1, caractérisé par l'hydrolyse des 17-(2-$OR_5$ 3-oxazolin-4-yl) delta$^{16}$-stéroïdes selon la revendication 2.

**11.** Procédé selon la revendication 1 ou 10, caractérisé en ce que l'hydrolyse s'effectue dans un solvant organique au moyen d'une solution aqueuse acide.

**12.** Procédé selon les revendications 1 à 11, caractérisé en ce que le 17-(isocyano-sulfonylméthylène)-stéroïde est de formule générale:

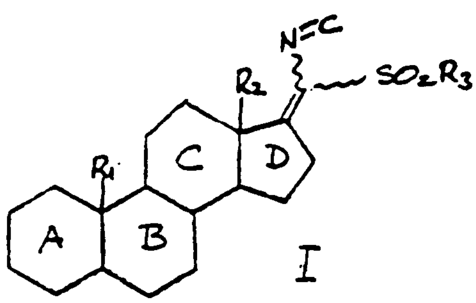

I

ou le 17-(2-$OR_5$ 3-oxazolin-4-yl) delta$^{16}$-stéroïde est de formule générale:

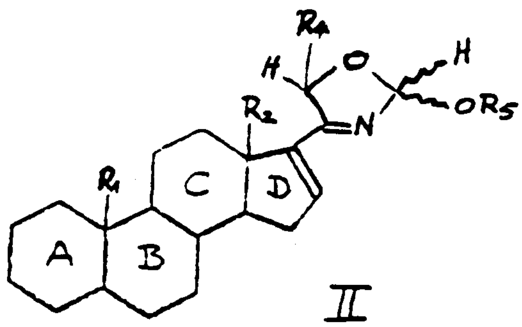

II

dans laquelle $R_1$ représente un atome d'hydrogène ou un radical méthyle ou bien est absent quand il existe une double liaison entre $C_{10}$ et $C_1$, $C_5$ ou $C_9$, $R_2$ représente un atome d'hydrogène ou un radical méthyle, $R_3$ représente un radical, soit alkyl soit dialkylamino, ou un radical aryle éventuellement substitué par un ou plusieurs atomes d'halogène ou radicaux alkyle, alkyloxy, nitro ou cyano, $R_4$ représente un atome d'hydrogène ou un radical alkyle comportant 1 à 4 atomes de carbone et éventuellement substitué par un ou plusieurs atomes d'halogène ou radical alkyloxy ou un radical phényle éventuellement substitué par un ou plusieurs atomes d'halogène ou radicaux alkyle ou alkyloxy, $R_5$ représente un radical alckyle ou arylalkyle, et les noyaux A, B, C et D contiennent éventuellement une ou plusieurs double liaisons, et sont éventuellement substitués par un ou plusieurs radicaux hydroxy, amino, atomes d'oxygène ou d'halogène, ou radicaux alkyle, alkylène, alkyloxy ou alkyloxyalkyloxy, et sont éventuellement disubstitués par un ou plusieurs radicaux époxy ou méthylène, radicaux alkylènedioxy, alkylènedithio ou alkylènoxythio.

**13.** Procédé selon les revendications 1 à 12, caractérisé en ce que $R_4$ représente un atome d'hydrogène et $R_5$ représente un radical méthyle.

**14.** Procédé tel que défini à la revendication 12 ou 13, caractérisé en ce que $R_3$ représente un radical alkyle comportant 1 à 10 atomes de carbone, ou $R_3$ représente un radical phényle ou naphtyle éventuellement substitué par un atome d'halogène ou un radical alkyloxy, ou par un ou plusieurs radicaux alkyle, $R_3$ représentant de préférence un radical phényle, p-méthoxyphényle ou p-méthylphényle.

**15.** Procédé selon les revendications 12 à 14 dans lequel les noyaux A, B, C et D contiennent une ou plusieurs double liaisons, caractérisé en ce que ces doubles liaisons sont présentes entre $C_1$et $C_2$, $C_3$ et $C_4$, $C_4$ et $C_5$, $C_5$ et $C_6$, $C_6$ et $C_7$, $C_9$ et $C_{11}$, et/ou $C_{11}$ et $C_{12}$.

**16.** Procédé selon les revendications 12 à 15, caractérisé en ce que les noyaux A, B, C et D sont substitués par un ou plusieurs radicaux 3-, 9-, 11-, 12- ou 14-hydroxy, et/ou par un atome d'oxygène à $C_3$, $C_{11}$ ou $C_{12}$, et/ou par un ou plusieurs atomes de 6-, 9- ou 11- fluor, chlore ou brome, et/ou par un radical 1- ou 6- méthyle, et/ou par un radical 3-, 9- ou 11- alkyloxy comportant 1 à 4 atomes de carbone, et/ou par un radical 3- ou 11- alkyloxyalkyloxy.

**17.** Procédé selon les revendications 12 à 16, caractérisé en ce que les noyaux A, B, C et D sont disubstitués par un radical époxy à $C_1$ et $C_2$ ou à $C_9$ et $C_{11}$ et/ou par un radical méthylène lié à $C_1$ et $C_2$ et/ou par un radical 3, 3-alkylènedioxy, 3,3-alkylènedithio ou 3,3-alkylènoxythio.

**18.** Les 17-(2-$OR_5$ 3-oxazolin-4-yl) $delta^{16}$-stéroïdes de formule II :

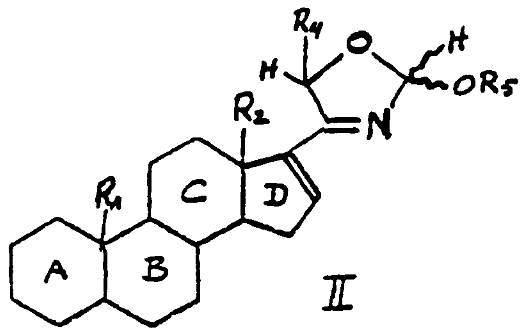

II

dans laquelle $R_1$ représente un atome d'hydrogène ou un radical méthyle ou bien est absent quand il existe une double liaison entre $C_{10}$ et $C_1$, $C_5$ ou $C_9$, $R_2$ représente un atome d'hydrogène ou un radical méthyle, $R_4$ représente un atome d'hydrogène ou un radical, soit alkyle soit phényle, $R_5$ représente un radical alkyle, et les noyaux A, B, C et D contiennent éventuellement une ou plusieurs double liaisons, et sont éventuellement substitués par un ou plusieurs radicaux, hydroxy, amino, atomes d'oxygène ou d'halogène, ou radicaux alkyle, alkylène, alkyoxy ou alkyloxyalkylolxy, et sont éventuellement disubstitués par un ou plusieurs radicaux époxy, méthylène ou alkylènedioxy, alkylènedithio, ou alkylènoxythio.

### Patentansprüche

**1.** Verfahren zur Herstellung von 21-Hydroxy-20-keto-$delta^{16}$-steroiden der Formel III

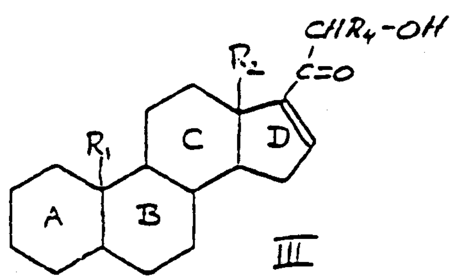

III

worin $R_1$ ein Wasserstoffatom oder eine Alkylgruppe mit 1 bis 4 Kohlenstoffatomen bedeutet oder, wenn zwischen $C_{10}$ und $C_1$, $C_5$ oder $C_9$ eine Doppelbindung vorliegt, abwesend ist,
$R_2$ ein Wasserstoffatom oder eine Alkylgruppe mit 1 bis 4 Kohlenstoffatomen bedeutet,
$R_4$ ein Wasserstoffatom oder eine Alkylgruppe mit 1 bis 4 Kohlenstoffatomen, welche gegebenenfalls durch ein oder mehrere Halogenatome oder Alkoxygruppen substituiert ist, oder eine Phenylgruppe, welche gegebenenfalls durch ein oder mehrere Halogenatome oder Alkyl- oder Alkoxygruppen substituiert ist, bedeutet und
die Ringe A, B, C und D eine oder mehrere Doppelbindungen enthalten können und substituiert sein können durch eine oder mehrere der folgenden Gruppen: Hydroxy, Amino, Oxo, Halogen, Alkyl, Alkylen, Alkoxy, Alkoxyalkoxy, Epoxy, Methylen, Alkylendioxy, Alkylendithio oder Alkylenoxythio, dadurch gekennzeichnet, daß man ein 17-(Isocyano-sulfonyl-methylen)-steroid der Formel I

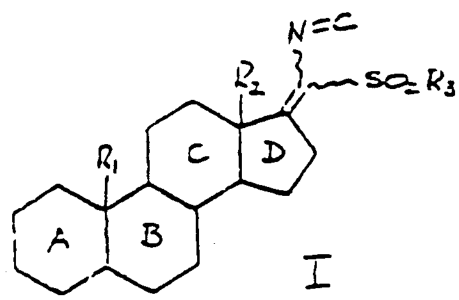

I

worin $R_1$ und $R_2$ die vorstehend angegebene Bedeutung besitzen und die Ringe A, B, C und D Doppelbindungen enthalten können und Substituenten, wie vorstehend definiert, tragen können und worin $R_3$ für eine Alkylgruppe eine Arylgruppe, die substituiert oder nicht substituiert ist durch ein oder mehrere Halogenatome oder Alkyl-, Alkoxy-, Nitro- oder Cyanogruppen, steht oder für eine Dialkylaminogruppe steht, worin die Alkylgruppen gemeinsam mit dem Stickstoffatom einen heterocyclischen Ring mit bis zu 8 Ringatomen,der gegebenenfalls ein Sauerstoffatom enthält, bilden können,unter basischen Bedingungen mit einem Aldehyd $R_4HO$ umsetzt, worin $R_4$ ein Wasserstoffatom oder eine Alkylgruppe mit 1 bis 4 Kohlenstoffatomen, die gegebenenfalls durch ein oder mehrere Halogenatome oder Alkoxygruppen substituiert ist, oder eine Phenylgruppe, die gegebenenfalls durch ein oder mehrere Halogenatome oder Alkyl- oder Alkoxygruppen substituiert ist, bedeutet
und mit einem Alkylalkohol oder einem Arylalkylalkohol umsetzt, woran sich eine Hydrolyse anschließt.

**2.** Verfahren zur Herstellung von 17-(2-$OR_5$-3-Oxazolin-4-yl)-delta$^{16}$-steroiden der Formel II

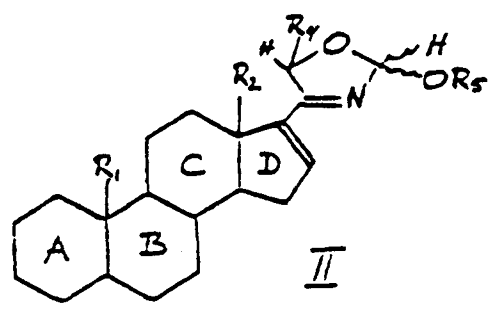

II

worin $R_1$, $R_2$ und $R_4$ die in Anspruch 1 angegebene Bedeutung besitzen, die Ringe A, B, C und D Doppelbindungen enthalten können und Substituenten, wie in Anspruch 1 definiert, haben können, und $R_5$ für eine Alkyl- oder Arylalkylgruppe steht, dadurch gekennzeichnet, daß man ein 17-(Isocyano-sulfonyl-methylen)-steroid gemäß Anspruch 1 unter basischen Bedingungen mit einem Aldehyd und einem Alkohol, wie in Anspruch 1 definiert, umsetzt.

**3.** Verfahren gemäß Anspruch 1 oder 2, dadurch gekennzeichnet, daß die Umsetzung in einem inerten organischen Lösungsmittel unter Verwendung eines Metallhydroxids, eines Metallalkoholats oder eines

quaternären Ammonium- oder Phosphoniumhydroxids als Base durchgeführt wird.

4. Verfahren gemäß Anspruch 3, dadurch gekennzeichnet, daß ein Katalysator zu der Reaktionsmischung in Form eines quaternären Ammonium- oder Phosphoniumsalzes oder eines Kronenäthers zugegeben wird.

5. Verfahren gemäß Anspruch 1 oder 2, dadurch gekennzeichnet, daß die Umsetzung unter Phasentransferbedingungen durchgeführt wird.

6. Verfahren gemäß den Ansprüchen 1 bis 5, dadurch gekennzeichnet, daß der Aldehyd ein Phenylaldehyd, worin die Phenylgruppe gegebenenfalls durch ein oder mehrere Halogenatome oder Alkyl- oder Alkoxygruppen substituiert ist,oder ein Alkylaldehyd, worin die Alkylgruppe 1 bis 4 Kohlenstoffatome aufweist und gegebenenfalls durch ein oder mehrere Halogenatome oder Alkoxygruppen substituiert ist, vorzugsweise Benzaldehyd oder Acetaldehyd ist.

7. Verfahren gemäß den Ansprüchen 1 bis 6, dadurch gekennzeichnet, daß der Aldehyd Formaldehyd ist.

8. Verfahren gemäß den Ansprüchen 1 bis 7, dadurch gekennzeichnet, daß der Alkohol ein Alkylalkohol oder Arylalkylalkohol, vorzugsweise ein $C_1$-$C_6$-Alkylalkohol ist.

9. Verfahren gemäß den Ansprüchen 1 bis 8, dadurch gekennzeichnet, daß der Alkohol Methanol oder Ethanol ist.

10. Verfahren zur Herstellung von 21-Hydroxy-20-keto-delta$^{16}$-steroiden gemäß Anspruch 1, gekennzeichnet durch die Hydrolyse von 17-(2-$OR_5$-3-Oxazolin-4-yl)-delta$^{16}$-steroiden gemäß Anspruch 2.

11. Verfahren gemäß Anspruch 1 oder 10, dadurch gekennzeichnet, daß die Hydrolyse in einem organischen Lösungsmittel unter Verwendung einer sauren wäßrigen Lösung durchgeführt wird.

12. Verfahren gemäß den Ansprüchen 1 bis 11, dadurch gekennzeichnet, daß das 17-(Isocyano-sulfonylmethylen)-steroid die allgemeine Formel

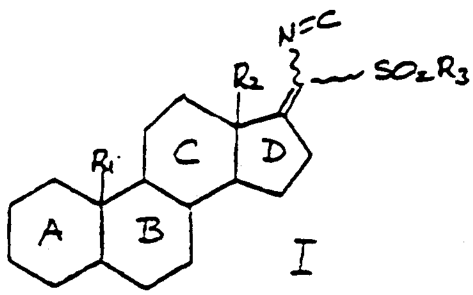

I

besitzt oder das 17-(2-$OR_5$-3-Oxazolin-4-yl)-delta$^{16}$-steroid die allgemeine Formel

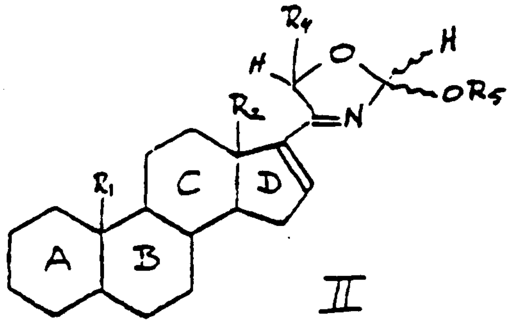

II

besitzt, worin $R_1$ für ein Wasserstoffatom oder eine Methylgruppe steht, oder, wenn eine Doppelbindung zwischen $C_{10}$ und $C_1$, $C_5$ oder $C_9$ vorliegt, abwesend ist, $R_2$ ein Wasserstoffatom oder eine Methylgruppe bedeutet, $R_3$ eine Alkyl- oder Dialkylaminogruppe oder eine Arylgruppe, welche substituiert oder nicht substituiert ist durch ein oder mehrere Halogenatome, Alkyl-, Alkoxy-, Nitro oder Cyanogruppen, bedeutet, $R_4$ für ein Wasserstoffatom oder eine Alkylgruppe mit 1 bis 4 Kohlenstoffatomen, welche gegebenenfalls durch ein oder mehrere Halogenatome oder Alkoxygruppen substituiert ist,oder eine Phenylgruppe, welche gegebenenfalls durch ein oder mehrere Halogenatome oder Alkyl- oder Alkoxygruppen substituiert ist, bedeutet, $R_5$ für eine Alkyl- oder Arylalkylgruppe steht und die Ringe A, B, C und D gegebenenfalls eine oder mehrere Doppelbindungen enthalten und gegebenenfalls substituiert sind durch eine oder mehrere Hydroxygruppen, Aminogruppen, Sauerstoffatome, Halogenatome oder Alkyl-, Alkylen-, Alkoxy- oder Alkoxyalkoxygruppen und disubstituiert oder nicht disubstituiert sind durch eine oder mehrere Epoxygruppen, Methylengruppen oder Alkylendioxy-, Alkylendithio- oder Alkylenoxythiogruppen.

**13.** Verfahren gemäß den Ansprüchen 1 bis 12, dadurch gekennzeichnet, daß $R_4$ für ein Wasserstoffatom steht und $R_5$ eine Methylgruppe bedeutet.

**14.** Verfahren gemäß Anspruch 12 oder 13, dadurch gekennzeichnet, daß $R_3$ für eine Alkylgruppe mit 1 bis 10 Kohlenstoffatomen steht oder $R_3$ eine Phenyl- oder Naphthylgruppe, gegebenenfalls substituiert durch ein Halogenatom, eine oder mehrere Alkylgruppen oder eine Alkoxygruppe,bedeutet, vorzugsweise $R_3$ für eine Phenyl-, p-Methoxyphenyl- oder p-Methylphenylgruppe steht.

**15.** Verfahren gemäß den Ansprüchen 12 bis 14, worin die Ringe A, B, C und D eine oder mehrere Doppelbindungen enthalten, dadurch gekennzeichnet, daß diese Doppelbindungen zwischen $C_1$ und $C_2$, $C_3$ und $C_4$, $C_4$ und $C_5$, $C_5$ und $C_6$, $C_6$ und $C_7$, $C_9$ und $C_{11}$ und/oder $C_{11}$ und $C_{12}$ anwesend sind.

**16.** Verfahren gemäß den Ansprüchen 12 bis 15, dadurch gekennzeichnet, daß die Ringe A, B, C und D durch eine oder mehrere 3-, 9-, 11-, 12- oder 14-Hydroxygruppen und/oder durch ein Sauerstoffatom in $C_3$, $C_{11}$ oder $C_{12}$ und/oder durch ein oder mehrere 6-, 9- oder 11-Fluor-, Chlor- oder Bromatome und/oder durch eine 1 oder 6-Methylgruppe und/oder durch eine 3-, 9- oder 11-Alkoxygruppe, die 1 bis 4 Kohlenstoffatome enthält, und/oder durch eine 3- oder 11-Alkoxyalkoxygruppe substituiert sind.

**17.** Verfahren gemäß den Ansprüchen 12 bis 16, dadurch gekennzeichnet, daß die Ringe A, B, C und D durch eine Epoxygruppe in $C_1$ und $C_2$ oder $C_9$ und $C_{11}$ und/oder durch eine an $C_1$ und $C_2$ gebundene Methylengruppe und/oder durch eine 3,3-Alkylendioxy-, eine 3,3-Alkylendithio- oder 3,3-Alkylenoxythiogruppe disubstituiert sind.

**18.** 17-(2-$OR_5$-3-Oxazolin-4-yl)-delta$^{16}$-steroidemit der allgemeinen Formel

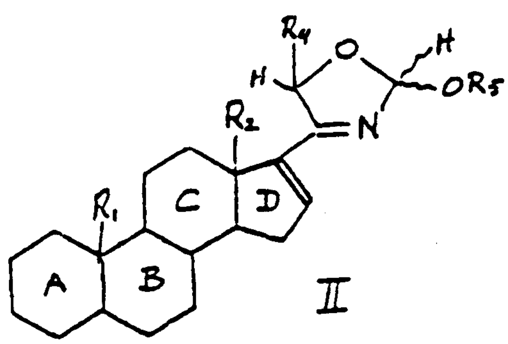

II

worin $R_1$ ein Wasserstoffatom oder eine Methylgruppe bedeutet oder, im Fall einer Doppelbindung zwischen $C_{10}$ und $C_1$, $C_5$ oder $C_9$, abwesend ist, $R_2$ ein Wasserstoffatom oder eine Methylgruppe darstellt, $R_4$ ein Wasserstoffatom oder eine Alkyl- oder eine Phenylgruppe bedeutet, $R_5$ für eine Alkylgruppe steht und die Ringe A, B, C und D gegebenenfalls eine oder mehrere Doppelbindungen enthalten, und gegebenenfalls durch eine oder mehrere Hydroxygruppen, Aminogruppen, Sauerstoffatome, Halogenatome oder Alkyl-, Alkylen-, Alkoxy-, oder Alkoxyalkoxygruppen substituiert sind und

disubstituiert oder nicht disubstituiert sind durch eine oder mehrere Epoxygruppen, Methylengruppen oder Alkylendioxy-, Alkylendithio- oder Alkylenoxythiogruppen.